(19) Europäisches Patentamt
European Patent Office
Office européen des brevets

(11) Veröffentlichungsnummer: **0 302 503 B1**

(12) # EUROPÄISCHE PATENTSCHRIFT

(45) Veröffentlichungstag der Patentschrift: **20.05.92**

(51) Int. Cl.$^5$: **A61K 37/54**

(21) Anmeldenummer: **88112737.7**

(22) Anmeldetag: **04.08.88**

(54) **Verfahren zur Abtrennung und Reinigung von t-PA.**

(30) Priorität: **05.08.87 DE 3726019**

(43) Veröffentlichungstag der Anmeldung:
**08.02.89 Patentblatt 89/06**

(45) Bekanntmachung des Hinweises auf die Patenterteilung:
**20.05.92 Patentblatt 92/21**

(84) Benannte Vertragsstaaten:
**AT BE CH DE ES FR GB GR IT LI LU NL SE**

(56) Entgegenhaltungen:
**EP-A- 0 124 613**
**EP-A- 0 163 751**
**EP-A- 0 178 105**
**EP-A- 0 236 289**

**BIOLOGICAL ABSTRACTS, Band 71, Nr. 7, 1981, Zusammenfassung Nr. 47459, Biological Abstracts Inc., Philadelphia, PA, US; Y.-C. SU et al.: "Purification of urokinase by affinity column chromatography", && NATL. SCI. COUNC. MON. 8(5): 393-405, 1980**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Macartney, Henry W., Dr. rer.nat.**
**Kirchenweg 6**
**W-8121 Sindelsdorf(DE)**
Erfinder: **Menhart, Josef**
**In der Au 15**
**W-8120 Weilheim(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 302 503 B1

Rank Xerox (UK) Business Services
(3.08/2.19/2.0)

## Beschreibung

Die Erfindung betrifft ein Verfahren zur Abtrennung und Reinigung von t-PA aus einer t-PA enthaltenden wäßrigen Proteinlösung durch Affinitätschromatographie an einem farbstoffhaltigen unlöslichen Kohlehydrat.

Die Herstellung von t-PA (tissue-Plasminogen Activator) hat aufgrund der gerinnselauflösenden Wirkung von t-PA und deren Möglichkeiten zur Behandlung von Herzkoronarerkrankungen in der letzten Zeit große Bedeutung erlangt. So gelingt es aus verschiedenen Zellinien z. B. menschlichen Melanomzellen, oder CHO (Chinese Hamster Ovary) Zellen t-PA enthaltende wäßrige Proteinlösungen zu erhalten. Die Möglichkeiten zur Reinigung, Abtrennung und Reinigung des Proteins von anderen zellulären Proteinen sind bisher jedoch noch nicht zufriedenstellend, unter anderem deshalb, weil t-PA nur in Spuren in solchen Zellen vorhanden ist.

So wird in der EP 178 105 A2 und in Biochemica et Biophysica Acta 704 (1982) 450-460 ein Verfahren zur Reinigung von t-PA über Affinitätschromatographie mit blauen Triazinfarbstoffen beschrieben. Hierbei ist ein Aufreinigungsfaktor von ca. 20 bis 30 erreicht worden. In "Kontakte" (Darmstadt) 1984 (2), Seite 32 bis 40 wird die Affinitätschromatographie von Proteinen mit Triazinfarbstoffen im allgemeinen beschrieben. Um eine ausreichende Affinität zu Triazinfarbstoffen zu erzielen, müssen Proteine jedoch bestimmte Coenzyme aufweisen und der jeweils geeignete Triazinfarbstoff soll mit dem jeweiligen Coenzym übereinstimmende Strukturmerkmale besitzen. Solche Coenzyme sind NAD, NADP, COA, FAD und Folsäure. Da t-PA kein Coenzym aufweist, schien eine Reinigung über solche Farbstoffe nicht erfolgversprechend.

Eine gute Anreicherung von t-PA gelingt unter Verwendung einer Affinitätschromatographie, bei der monoklonale Antikörper, die gegen t-PA gerichtet sind, an einem Säulenmaterial immobilisiert vorliegen (EMBO Journal 2 (1983) 115-119 und BBA 830 (1985) 1-10). Ein weiteres Verfahren besteht darin, an Sepharose gekoppeltes Fibrin zur Affinitätschromatographie zu verwenden (BBA 621 (1980) 241-254), da t-PA eine hohe Affinität zu Fibrin aufweist. Die beiden letzten Chromatographiematerialien sind jedoch teuer und umständlich in ihrer Herstellung.

Darüberhinaus sind sie bei der Verwendung von z. B. CHO-Zellüberständen als aufzuarbeitendes Material nicht stabil. Insbesondere lösen im Überstand enthaltene Proteasen das Fibrin und die Antikörper zum Teil ab. Dadurch wird die Säule zerstört, was zum einen die Dauer der Einsetzbarkeit begrenzt und zum zweiten störende Proteine im Eluat erscheinen läßt.

Außerdem gelingt es mit keinem dieser Chromatographiematerialien, t-PA aus dem in CHO-Überständen ebenfalls enthaltenen Inhibitor-t-PA-Komplex freizusetzen (ca. 60% der t-PA-Aktivität ist komplexiert).

Die Aufgabe der vorliegenden Erfindung besteht demnach darin, ein Verfahren zur Reinigung von t-PA bereitzustellen, mit dem eine hohe Anreicherung der Aktivität unter Verwendung eines kostengünstigen, einfach herzustellenden und stabilen Chromatographiematerials möglich ist. Außerdem soll eine Freisetzung von t-PA aus den t-PA-Inhibitor-Komplexen erreicht werden.

Erfindungsgemäß wird diese Aufgabe gelöst durch ein Verfahren zur Abtrennung und Reinigung von t-PA aus einer t-PA enthaltenden wäßrigen Proteinlösung durch Affinitätschromatographie an einem farbstoffhaltigen unlöslichen Kohlehydrat, das dadurch gekennzeichnet ist, daß man für die Chromatographie ein Kohlenhydrat verwendet, welches mit einem Farbstoff der allgemeinen Formel

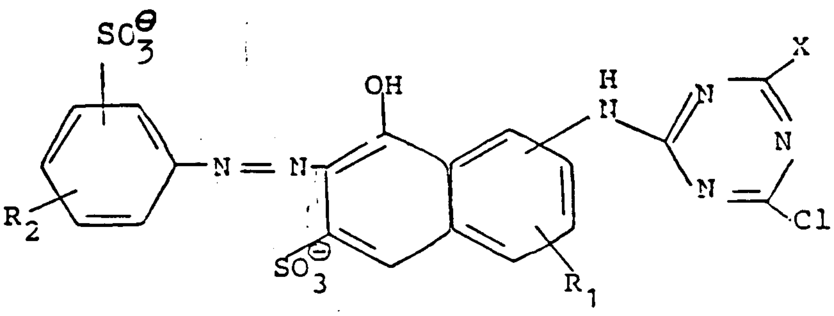

in der X = -Cl oder

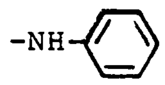 ,

$R_1$ = -H oder $-SO_3^{\ominus}$ und $R_2$ = -H, -OH oder $-OCH_3$ bedeutet, substituiert ist und das t-PA aus einer gepufferten Lösung bei einem pH-Wert von 5,5 bis 8,5 aufzieht.

Durch dieses Verfahren gelingt es überraschenderweise, t-PA aus beliebigen Quellen, z. B. Rifkin Melanomazellen, CHO-Zellen, reoxidiertes t-PA aus E. coli usw., mit überlegenen Resultaten zu reinigen. Der Anreicherungsfaktor für die Reinigung von t-PA aus CHO-Überständen liegt dabei zwischen 70- und 200fach, abhängig von der spezifischen Aktivität des Ausgangsmaterials.

Im natürlichen Material sind t-PA- Inhibitoren vorhanden, die mit t-PA auch Komplexe bilden. In dieser Form ist die biologische Aktivität von t-PA unterbunden oder jedenfalls stark herabgesetzt. Ein überraschender Effekt des Verfahrens der Erfindung ist nun, daß während dieser Chromatographie die Inhibitorkomplexe verschwinden und daher im Eluat bei solchem Inhibitorkomplex enthaltendem Ausgangsmaterial eine Steigerung der gemessenen t-PA-Aktivität von bis zu 30% gegenüber dem Ausgangsmaterial festzustellen ist, zurückzuführen auf das Verschwinden der Hemmung. Erfindungsgemäß gelingt es, bei t-PA, das ja kein Coenzym enthält, durch Farbstoffchromatographie ebenso gute Reinigungsresultate zu erzielen, wie mit Chromatographie an einer monoklonale Antikörper gegen t-PA tragenden Säule. Dies ist insofern erstaunlich, da man für die Anreicherung eines Enzyms an einer unspezifischen Matrix - in diesem Fall Farbstoff gekoppelt an Agarose - keine vergleichbare Anreicherung wie mit einer spezifischen Matrix, z. B. monoklonale Antikörper gegen t-PA, erwarten kann.

Bevorzugt werden als Farbstoffe Verbindungen der allgemeinen Formel mit X = -Cl, $R_1$ = $-SO_3^{\ominus}$ und $R_2$ = -H oder -OH. Ebenfalls bevorzugt sind die Verbindungen mit X = -Cl, $R_1$ = -H und $R_2$ = -H oder -OH sowie mit

X = -NH- 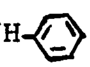

(Anilid), $R_1$ = $-SO_3^{\ominus}$ und $R_2$ = -H. Wenn Anilid, können auch zwei dieser Verbindungen über dessen Benzolring verbunden sein, derart, daß das Anilid zum p-Phenylendiamid ergänzt wird.

Besonders bevorzugt wird als Farbstoff eine Verbindung verwendet, bei der X = -Cl, $R_1$ = $-SO_3^{\ominus}$ und $R_2$ = -H ist. Eine solche Verbindung ist unter der Bezeichnung Procion® Red-MX-2B von der Firma ICI erhältlich.

Als Chromatographieträgermaterialien sind alle unlöslichen Kohlehydrate, die zu Chromatographiezwekken verwendet werden können, geeignet. Bevorzugt verwendet man als Kohlehydrat Stärke, Cellulose oder Agarose, wobei Agarosen, wie z. B. die unter der Bezeichnung Sepharose® vertriebenen, besonders bevorzugt sind.

Besonders gute Ergebnisse bei der Reinigung von t-PA werden mit einem Chromatographiematerial erhalten, bei dem Procion Red-MX-2B an Agarose gebunden ist.

Bevorzugt ist bei dem erfindungsgemäßen Verfahren der Farbstoff in einer Menge von 0,5 bis 10 Gew.-%, bezogen auf die Menge an Kohlehydrat vorhanden. Besonders bevorzugt liegt diese Menge zwischen 1 und 5 Gew.-% der Menge an Kohlehydrat.

Die Aufziehlösung enthält als Neutralsalz hoher Ionenstärke ein Alkalihalogenid oder ein anderes Alkalisalz einer Säure von etwa gleicher Ionenstärke in der gepufferten Lösung. In einer besonders bevorzugten Ausgestaltung der Erfindung werden bei der Chromatographie als Aufzieh- oder/und Elutionslösungen gepufferte Kaliumsalzlösungen verwendet. Solche Kaliumsalzlösungen haben den unerwarteten, aber bedeutsamen Vorteil, daß mit ihnen stets klare Lösungen erhalten werden, wogegen bei Verwendung anderer Salzlösungen Trübungen auftreten können. Noch mehr bevorzugt verwendet man eine Kaliumchloridlösung als Aufziehlösung. Die Neutralsalzkonzentration der Aufziehlösung läßt sich für jedes Ausgangsmaterial durch wenige Vorproben leicht herausfinden. Im allgemeinen sind Konzentrationen zwischen etwa 0,1 und 2 mol/l gut geeignet.

Der pH-Wert der Aufziehlösung beträgt 5,5 bis 8,5, bevorzugt 7 bis 8. Zum Puffern dieser Lösung eignen sich die in diesem Bereich wirksamen Puffersubstanzen wie z. B. Kaliumphosphatpuffer, Natriumphosphatpuffer, Zitratpuffer oder Trispuffer. Bevorzugt wird ein Kaliumphosphatpuffer verwendet. In einer weiteren Ausgestaltung der Erfindung enthält die Elutionslösung auch 0 bis 2 mol/l KSCN.

Die Elution von an die Säule gebundenem t-PA wird im erfindungsgemäßen Verfahren bevorzugt mit einem abnehmenden Gradienten des zum Aufziehen verwendeten Salzes, beginnend mit der Aufziehkonzentration, und mit gleichzeitig zunehmendem Gradienten von KSCN durchgeführt. Dabei kann die Elution mit kontinuierlich zu- oder abnehmenden Gradienten, oder aber auch mit schrittweise zu- bzw. abnehmenden Gradienten durchgeführt werden. Bei der schrittweisen Elution wird bevorzugt mit Konzentrationssprüngen von zwischen 0,05 mol/l und 0,2 mol/l für die Bestandteile der Elutionslösung gearbeitet.

Bevorzugt wird mit einem Überschuß von 10 bis 50 % an Säulenkapazität in Bezug auf das im zu reinigenden Material enthaltene fixierbare t-PA gearbeitet. Bei dem nach den Beispielen hergestellten 2,2-% Farbstoff enthaltenden Kohlenhydrat ergab sich eine Kapazität von 0,03 mg t-PA pro ml äquilibriertem Chromatographiematerial, also der in gequollenem Zustand zur Verwendung bereiten Säule.

Die folgenden Beispiele sollen die Erfindung weiter erläutern:

**Beispiel 1**

Herstellung des Chromatographiematerials.

100 g abgenutschte gewaschene Agarose (Sepharose CL-6B) wurden in 400 ml $H_2O$ suspendiert und unter Rühren auf 40 °C erhitzt. Anschließend wurde 2,2 g des Farbstoffs der allgemeinen Formel mit X = -Cl, $R_1$ = $-SO_3^{\ominus}$ und $R_2$ = -H (Procion Red MX-2B), suspendiert in 30 ml $H_2O$ zugegeben. Nach 10 Minuten wurden 40 ml 4 M NaCl zugegeben und nach weiteren 30 Minuten mit 1N NaOH auf eine Konzentration von 0,1N NaOH eingestellt. Bei 40 °C (± 5 °C) wurde 72 Stunden lang vorsichtig gerührt. Nach Abnutschen wurde in der angegebenen Reihenfolge mit folgenden Puffern gewaschen: $H_2O$, 5 M Harnstoff, $H_2O$, 2 M NaCl, $H_2O$, HCl. Das Produkt ist danach gebrauchsfertig und wird nachstehend als "Red-S" bezeichnet.

**Beispiel 2**

Reinigung von CHO-t-PA-Kulturüberständen

a)

| Reinigungsschema | Vol (ml) | Aktivität % | Protein % | Anreicherung |
|---|---|---|---|---|
| 1. Ausgangslösung | 1720 | 100 | 100 | - |
| 2. Red S-Eluat | 1225 | 71,3 | 0,74 | 96,5-fach |

b)

| Reinigungsschema | Vol (ml) | Aktivität % | Protein % | Anreicherung |
|---|---|---|---|---|
| 1. Ausgangslösung | 270 | 100 | 100 | - |
| 2. Red S-Eluat | 48 | 122 | 0,7 | 174-fach |

c)

| Reinigungsschema | Vol (ml) | Aktivität % | Protein % | Anreicherung |
|---|---|---|---|---|
| 1. Ausgangslösung | - | 100 | 100 | - |
| 2. Red S-Eluat | - | 64 | 0,7 | 91-fach |

Die Kapazitäten und Flußraten für die Beispiele 2a) bis c) sind ähnlich. Bei allen drei Beispielen sind die Anreicherungsfaktoren ebenfalls ähnlich. Die Flußraten betrugen für das Aufziehen von Kulturüberstand auf Red S 90 ml/h, für das Waschen und Eluieren 500 ml/h. Als Äquilibrationspuffer wurden 200 mmol Kaliumphosphat, pH 7,5, 0,01 % Tween 80 verwendet, als Wasch- bzw. Eluationspuffer wurde der Äquilibrationspuffer mit zunehmenden Gradienten von KSCN (0 bis 2 M) und abnehmenden Gradienten von NaCl (2 bis 0 M) verwendet.

**Patentansprüche**

**1.** Verfahren zur Abtrennung und Reinigung von t-PA aus einer t-PA enthaltenden wäßrigen Proteinlösung durch Affinitätschromatographie an einem farbstoffhaltigen unlöslichen Kohlehydrat, **dadurch gekennzeichnet**, daß man für die Chromatographie ein Kohlenhydrat verwendet, welches mit einem Farbstoff

der allgemeinen Formel

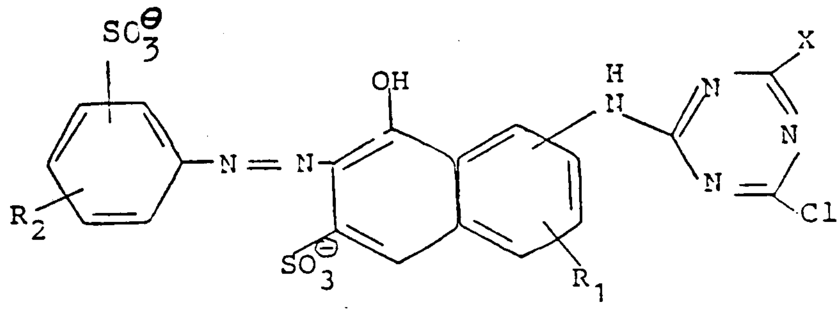

in der X = -Cl oder

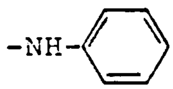

$R_1$ = -H oder $-SO_3^{\ominus}$ und $R_2$ = -H, -OH oder $-OCH_3$ bedeutet, substituiert ist und das t-PA aus einer gepufferten Lösung bei einem pH-Wert von 5,5 bis 8,5 aufzieht.

**2.** Verfahren nach Anspruch 1, **dadurch gekennzeichnet**, daß X = -Cl, $R_1$ = $-SO_3^{\ominus}$ und $R_2$ = H oder -OH ist.

**3.** Verfahren nach Anspruch 1, **dadurch gekennzeichnet**, daß X = -Cl, $R_1$ = -H und $R_2$ = -H oder -OH ist.

**4.** Verfahren nach Anspruch 1, **dadurch gekennzeichnet**, daß X =

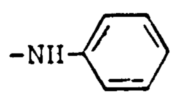

$R_1$ = $-SO_3^{\ominus}$ und $R_2$ = -H ist.

**5.** Verfahren nach Anspruch 1, **dadurch gekennzeichnet**, daß 2 Verbindungen der allgemeinen Formel von Anspruch 4 über eine p-Phenylendiamingruppe als Substituent X verbunden vorliegen.

**6.** Verfahren nach einem der Ansprüche 1 bis 5, **dadurch gekennzeichnet**, daß man als Kohlehydrat Stärke, Cellulose oder Agarose verwendet.

**7.** Verfahren nach Anspruch 6, **dadurch gekennzeichnet**, daß man Agarose verwendet.

**8.** Verfahren nach einem der Ansprüche 1 bis 7, **dadurch gekennzeichnet**, daß der Farbstoff in einer Menge von 0,5 bis 10 % Gew.-%, bezogen auf die Menge an Kohlehydrat, vorhanden ist.

**9.** Verfahren nach Anspruch 8, **dadurch gekennzeichnet**, daß der Farbstoff in einer Menge von 1 bis 5 Gew.-%, bezogen auf die Menge an Kohlehydrat, vorhanden ist.

**10.** Verfahren nach Anspruch 1, **dadurch gekennzeichnet**, daß der pH-Wert 7 bis 8 beträgt.

**11.** Verfahren nach einem der Ansprüche 1 bis 10, **dadurch gekennzeichnet**, daß man eine Aufziehlösung, die 0,1 bis 2 mol/l eines Alkalihalogenids oder eines anderen Alkalisalzes einer Säure von vergleichbarer Ionenstärke in gepufferter Lösung verwendet.

**12.** Verfahren nach einem der Ansprüche 1 bis 11, **dadurch gekennzeichnet**, daß man als Aufziehlösung eine gepufferte Kaliumsalzlösung verwendet.

**13.** Verfahren nach Anspruch 12, **dadurch gekennzeichnet**, daß man als Aufziehlösung eine KCl-Lösung verwendet.

**14.** Verfahren nach einem der Ansprüche 11 und 12, **dadurch gekennzeichnet**, daß man einen Kaliumphosphatpuffer verwendet.

**15.** Verfahren nach einem der vorhergehenden Ansprüche, **dadurch gekennzeichnet**, daß man zur Elution einen abnehmenden Gradienten des Alkalisalzes der Aufziehlösung verwendet.

**16.** Verfahren nach Anspruch 15, **dadurch gekennzeichnet**, daß die Elutionslösung 0 bis 2 mol/l KSCN enthält.

**17.** Verfahren nach Anspruch 15 und 16, **dadurch gekennzeichnet**, daß die Elution mit einem abnehmenden Gradienten des zum Aufziehen verwendeten Salzes, beginnend mit der Aufziehkonzentration, und mit einem zunehmenden Gradienten von KSCN durchgeführt wird.

**18.** Verfahren nach Anspruch 17, **dadurch gekennzeichnet**, daß die Elution mit kontinuierlich zu- bzw. abnehmenden Gradienten durchgeführt wird.

**19.** Verfahren nach Anspruch 17, **dadurch gekennzeichnet**, daß die Elution mit schrittweise zu- bzw. abnehmenden Gradienten durchgeführt wird.

**20.** Verfahren nach Anspruch 19, **dadurch gekennzeichnet**, daß die schrittweise Elution mit Konzentrationssprüngen von 0,05 mol/l bis 0,2 mol/l durchgeführt wird.

**Claims**

**1.** Process for the separation and purification of t-PA from an aqueous protein solution containing t-PA by affinity chromatography on an insoluble carbohydrate containing a coloured material, characterised in that, for the chromatography, one uses a carbohydrate which is substituted with a coloured material of the general formula:

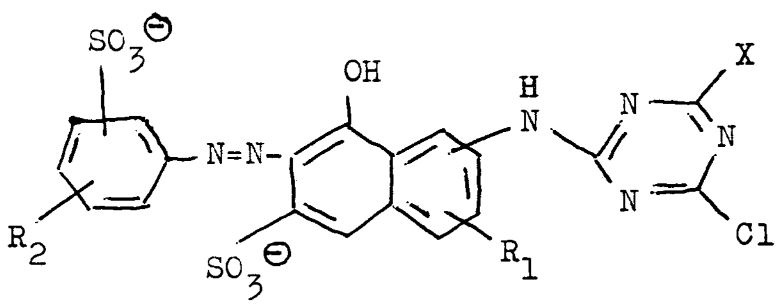

in which X = -Cl or

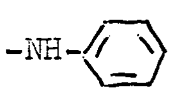 ,

$R_1$ = -H or $-SO_3^{\ominus}$ and $R_2$ = -H, -OH or $-OCH_3$, and absorbs the t-PA from the buffered solution with a pH value of 5.5 to 8.5.

**2.** Process according to claim 1, characterised in that X = -Cl, $R_1$ = $-SO_3^{\ominus}$ and $R_2$ = -H or -OH.

**3.** Process according to claim 1, characterised in that X = -Cl, $R_1$ - -H and $R_2$ = -H or -OH.

**4.** Process according to claim 1, characterised in that

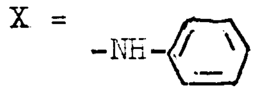

,

$R_1$ = $-SO_3^{\ominus}$ and $R_2$ = -H.

**5.** Process according to claim 1, characterised in that 2 compounds of the general formula of claim 4 are present bound via a p-phenylenediamine group as substituent X.

**6.** Process according to one of claims 1 to 5, characterised in that one uses starch, cellulose or agarose as carbohydrate.

**7.** Process according to claim 6, characterised in that one uses agarose.

**8.** Process according to one of claims 1 to 7, characterised in that the coloured material is present in an amount of 0.5 to 10 wt.%, referred to the amount of carbohydrate.

**9.** Process according to claim 8, characterised in that the coloured material is present in an amount of 1 to 5 wt.%, referred to the amount of carbohydrate.

**10.** Process according to claim 1, characterised in that the pH value amounts to 7 to 8.

**11.** Process according to one of claims 1 to 10, characterised in that one uses an absorption solution which contains 0.1 to 2 mol/l of an alkali metal halide or of another alkali metal salt of an acid of comparable ionic strength in buffered solution.

**12.** Process according to one of claims 1 to 11, characterised in that one uses a buffered potassium salt s olution as absorption solution.

**13.** Process according to claim 12, characterised in that one uses KCl solution as absorption solution.

**14.** Process according to one of claims 11 and 12, characterised in that one uses potassium phosphate buffer.

**15.** Process according to one of the preceding claims, characterised in that one uses a decreasing gradient of the alkali metal salt of the absorption solution for the elution.

**16.** Process according to claim 15, characterised in that the elution solution contains 0 to 2 mol/l KSCN.

**17.** Process according to claim 15 and 16, characterised in that the elution is carried out with a decreasing gradient of the salt used for the absorption, beginning with the absorption concentration, and with an increasing gradient of KSCN.

**18.** Process according to claim 17, characterised in that the elution is carried out with continuously increasing or decreasing gradients.

**19.** Process according to claim 17, characterised in that the elution is carried out stepwise with increasing or decreasing gradients.

**20.** Process according to claim 19, characterised in that the stepwise elution is carried out with concentration jumps of 0.05 mol/l to 0.2 mol/l.

## Revendications

1. Procédé de séparation et de purification de t-PA à partir d'une solution protéique aqueuse contenant du t-PA par chromatographie d'affinité sur un hydrate de carbone insoluble contenant un colorant, caractérisé en ce que l'on utilise pour la chromatographie un hydrate de carbone qui est substitué par un colorant de formule générale

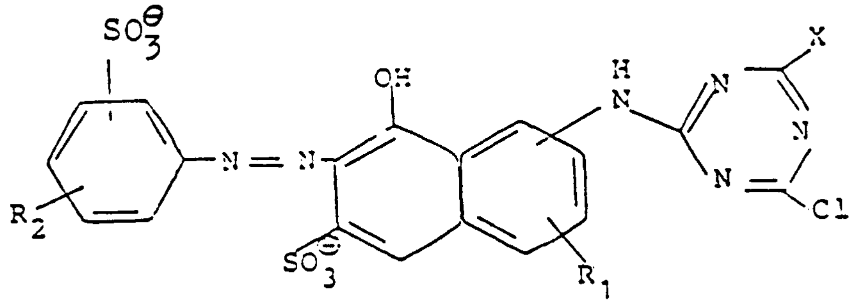

,

dans laquelle X = -Cl ou

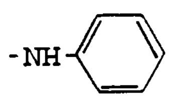

,

$R_1$ = -H ou $-SO_3^{\ominus}$ et $R_2$ = -H, -OH ou $-OCH_3$, et on fixe le t-PA à un pH de 5,5 à 8,5 à partir d'une solution tamponnée.

2. Procédé selon la revendication 1, caractérisé en ce que X = -Cl, $R_1$ = $-SO_3^{\ominus}$ et $R_2$ = -H ou -OH.

3. Procédé selon la revendication 1, caractérisé en ce que X = -Cl, $R_1$ = -H et $R_2$ = -H ou -OH.

4. Procédé selon la revendication 1, caractérisé en ce que

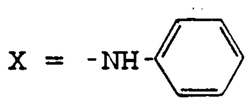

,

$R_1$ = $-SO_3^{\ominus}$ et $R_2$ = -H.

5. Procédé selon la revendication 1, caractérisé en ce que deux composés de formule générale selon la revendication 4 sont liés par un groupe p-phénylènediamine en tant que substituant X.

6. Procédé selon l'une des revendications 1 à 5, caractérisé en ce que l'on utilise comme hydrate de carbone l'amidon, la cellulose ou l'agarose.

7. Procédé selon la revendication 6, caractérisé en ce que l'on utilise l'agarose.

8. Procédé selon l'une des revendications 1 à 7, caractérisé en ce que le colorant est présent en une quantité de 0,5 à 10% en poids par rapport à la quantité d'hydrate de carbone.

9. Procédé selon la revendication 8, caractérisé en ce que le colorant est présent en une quantité de 1 à 5% en poids par rapport à la quantité d'hydrate de carbone.

10. Procédé selon la revendication 1, caractérisé en ce que le pH est de 7 à 8.

**11.** Procédé selon l'une des revendications 1 à 10, caractérisé en ce que l'on utilise une solution de fixation qui contient 0,1 à 2 mol/l d'un halogénure alcalin ou d'un autre sel alcalin d'un acide de force ionique comparable en solution tamponnée.

**12.** Procédé selon l'une des revendications 1 à 11, caractérisé en ce que l'on utilise comme solution de fixation une solution tamponnée de sel de potassium.

**13.** Procédé selon la revendication 12, caractérisé en ce que l'on utilise une solution de KCl comme solution de fixation.

**14.** Procédé selon l'une des revendications 11 et 12, caractérisé en ce que l'on utilise un tampon phosphate de potassium.

**15.** Procédé selon l'une des revendications précédentes, caractérisé en ce que l'on utilise pour l'élution un gradient décroissant du sel alcalin de la solution de fixation.

**16.** Procédé selon la revendication 15, caractérisé en ce que la solution d'élution contient 0 à 2 mol/l de KSCN.

**17.** Procédé selon les revendications 15 et 16, caractérisé en ce que l'élution est opérée avec un gradient décroissant du sel utilisé pour la fixation, en commençant avec la concentration de fixation, et avec un gradient croissant de KSCN.

**18.** Procédé selon la revendication 17, caractérisé en ce que l'élution est opérée avec des gradients continûment croissants ou décroissants.

**19.** Procédé selon la revendication 17, caractérisé en ce que l'élution est opérée avec des gradients croissants ou décroissants par degrés.

**20.** Procédé selon la revendication 19, caractérisé en ce que l'élution par degrés est opérée avec des sauts de concentration de 0,05 mol/l à 0,2 mol/l.